# EUROPEAN PATENT APPLICATION

(11) **EP 4 001 420 A2**
(43) Date of publication of application: **25.05.2022**
(21) Application number: 21209701.8
(22) Date of filing: 22.11.2021
(51) Int. Cl.: C12P 7/26, C12R 1/01, C12R 1/07, C12R 1/85

(54) **METHOD FOR PREPARING RASPBERRY KETONE USING INDUSTRIAL FERMENTATION**

(30) Priority: 20.11.2020 CN 202011316680
(71) Applicant: Xiamen Oamic Biotech Co., Ltd., Xiamen, Fujian 361026 (CN)
(72) Inventor: XING, Chenguang, Xiamen, 361026 (CN); ZHAO, Xijing, Xiamen, 361026 (CN); MIAO, Yangli, Xiamen, 361026 (CN); HONG, Ying, Xiamen, 361026 (CN); LIU, Wei, Xiamen, 361026 (CN)
(74) Representative: Verscht, Thomas Kurt Albert

(57) **Abstract**

The present disclosure is a method for preparing raspberry ketone using industrial fermentation, which comprises the following steps: (1) using natural p-coumaric acid as a raw material, metabolizing by *Actinomycetes* sp. OMK-74 to obtain p-hydroxybenzaldehyde; (2) using the p-hydroxybenzaldehyde obtained in step (1) and acetone as raw materials, metabolizing by *Bacillus* sp. OMK-75 to obtain 4-hydroxybenzyl acetone; and (3) using the 4-hydroxybenzyl acetone obtained in step (2) as a raw material, metabolizing by *Saccharomyces* sp. OMK-76 to be reduced to obtain the raspberry ketone. The present disclosure discloses a highly effective method for preparing raspberry ketone using biological synthesis by a three-step fermentation.

## Description

### FIELD OF THE DISCLOSURE

The present disclosure relates to a technical field for preparing raspberry ketone, and in particular relates to a method for preparing raspberry ketone using industrial fermentation.

### BACKGROUND OF THE DISCLOSURE

Raspberry ketone is also known as rubus ketone, and its chemical name is 4-p-hydroxyphenyl-2-butanone. Raspberry ketone is a main aroma component of raspberry. It is an elegantly fruity flavor and fragrance widely used in both inside and outside a country. It is widely used in cosmetics and foods. In addition, it can also be used to be synthesized into medicine.

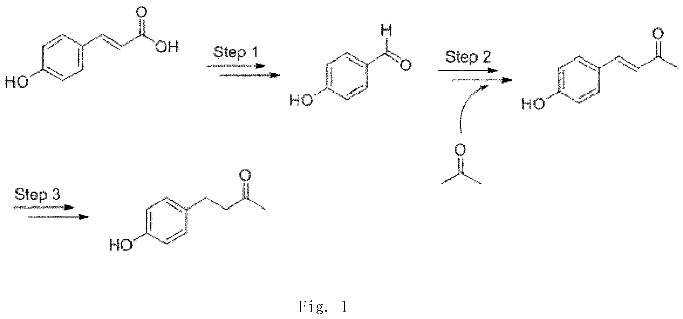

In existing techniques, the reports with respect to a method for synthesizing the raspberry ketone is as follows:
(1) In 1996, Claudio Fuganti et al. respectively used baker's yeast (Tetrahedron, 1996, 52. 4041-4052) and *Beauveria bassiana* (Tetrahedron: Asymmetry, 1996, 7, 3129-3134) to prepare raspberry ketone by reducing ethylenic bond (referring to Fig. 1) in 4-hydroxybenzylidene acetone, and an article was published in 1998 (Journal of Molecular Catalysis B: Enzymatic 1998, 4, 289-293) to screen microorganisms used to catalytically reduce the ethylenic bond again and investigate reduction products in detail. It was found that some microorganisms can selectively reduce the ethylenic bond, while most microorganisms reduced the ethylenic bond and a carbonyl group simultaneously to produce rhododendronol.
(2) In 1998, Whitehead, I.M. et al. (Food Technol 1998, 52, 40-46) used p-hydroxybenzaldehyde derived from fermention or plants and acetone derived from fermention to catalytically condensed by aldolase, 4-hydroxybenzylidene acetone is then obtained due to β-elimination reaction, and the raspberry ketone is finally obtained due to catalytically reducing the ethylenic bond by microorganisms.
(3) In 2007, G. Feron et al. (Letters in Applied Microbiology, 2007, 45, 29-35) used *Escherichia coli* as a genetic engineering host bacteria to express aldolase (DERA) derived from bacterial to achieve a condensation reaction between p-hydroxybenzaldehyde and acetone, and a final yield of 4-hydroxybenzylidene acetone functioning as a key intermediate in a synthesis of the raspberry ketone reached 160 mg/L due to process optimization.

Therefore, the aforementioned methods are still at a laboratory level, and a production of raspberry ketone in industrial scales cannot be achieved.

### BRIEF SUMMARY OF THE DISCLOSURE

An objective of the present disclosure is to overcome deficiencies of existing techniques and to provide a method for preparing raspberry ketone using industrial fermentation.

A technical solution of the present disclosure is as follows.

A method for preparing raspberry ketone using industrial fermentation, which comprises the following steps:
(1) using natural p-coumaric acid as a raw material, metabolizing by *Actinomycetes* sp. OMK-74 to obtain p-hydroxybenzaldehyde;
(2) using the p-hydroxybenzaldehyde obtained in step (1) and acetone as raw materials, metabolizing by *Bacillus* sp. OMK-75 to obtain 4-hydroxybenzyl acetone; and
(3) using the 4-hydroxybenzyl acetone obtained in step (2) as a raw material, metabolizing by *Saccharomyces* sp. OMK-76 to be reduced to obtain the raspberry ketone;

*Actinomycetes* sp. OMK-74 was deposited in China Center for Type Culture Collection on October 16, 2020, with a deposit number of CCTCC NO: M 2020584; *Bacillus* sp. OMK-75 was deposited in China Center for Type Culture Collection on October 16, 2020, with a deposit number of CCTCC NO: M 2020585; and *Saccharomyces* sp. OMK-76 was deposited in China Center for Type Culture Collection on October 16, 2020, with a deposit number of CCTCC NO: M2020586.

In a preferred embodiment of the present disclosure, the step (1) comprises:
A. taking *Actinomycetes* sp. OMK-74 refrigerated in glycerin, streaking on a plate, placing the plate at 36-37 °C, and cultivating until clones are full;
B. picking a single clone from the clones, inoculating into a first seed cultivating medium, cultivating at 36-37 °C and 180-220 rpm on a shaker or stirring and cultivating at 280-320 rpm in an aeration condition for 20-25 hours to obtain a first seed cultivating solution;
C. inoculating the first seed cultivating solution in a first fermentation medium with an inoculum amount of 8-12%, fermenting for 12-16 hours at 36-37 °C and pH=7.0-7.5 until OD₆₀₀ analyzed by a spectrophotometer no longer increases, adding p-coumaric acid at one time or several times until a final concentration is 48-52 g/L, then continually cultivating until a substrate is completely reacted or no longer continues to be reacted, and then purifying to obtain the p-hydroxybenzaldehyde.

In a further preferred embodiment, a composition of the first seed cultivating medium is shown in the following table:

| Product | Concentration (g/L) |
|---|---|
| malt extract powder | 30 |
| yeast powder | 15 |
| potassium dihydrogen phosphate | 1.2 |
| sodium chloride | 0.8 |
| pH | natural |

a solvent is water.

In a further preferred embodiment, a composition of the first fermentation medium is shown in the following table:

| Product | Concentration (g/L) |
|---|---|
| glucose | 30 |
| yeast powder | 0.8 |
| magnesium sulfate | 0.2 |
| potassium dihydrogen phosphate | 0.2 |
| sodium chloride | 0.25 |
| disodium phosphate | 0.5 |
| pH | natural |

a solvent is water.

In a preferred embodiment of the present disclosure, the step (2) comprises:
A. taking the *Bacillus* sp. OMK-75 refrigerated in glycerin, streaking on a plate, placing the plate at 29-30 °C, and cultivating until clones are full;
B. picking a single clone from the clones, inoculating into a second seed cultivating medium, cultivating at 29-30 °C and 180-220 rpm on a shaker or stirring and cultivating at 380-420 rpm in an aeration condition for 20-25 hours to obtain a second seed cultivating solution;
C. inoculating the second seed cultivating solution into a second fermentation medium with an inoculum amount of 8-12%, fermenting for 8-12 hours at 34-35 °C and pH=6.5-7.0 until OD₆₀₀ analyzed by a spectrophotometer no longer increases, adding p-hydroxybenzaldehyde and acetone at one time until final concentrations are respectively 38-42 g/L and 100-150 g/L, then continually cultivating until a substrate is completely reacted or no longer continues to be reacted, and then purifying to obtain the p-hydroxybenzylidene acetone.

In a further preferred embodiment, a composition of the second seed cultivating medium is shown in the following table:

| Product | Concentration (g/L) |
|---|---|
| glucose | 20 |
| yeast powder | 10 |
| peptone | 10 |
| dipotassium phosphate | 0.8 |
| sodium chloride | 0.8 |
| pH | natural |

a solvent is water.

In a further preferred embodiment, a composition of the second fermentation medium is shown in the following table:

| Product | Concentration (g/L) |
|---|---|
| glucose | 30 |
| yeast powder | 8.0 |
| peptone | 2.0 |
| magnesium sulfate | 0.2 |
| potassium dihydrogen phosphate | 1.0 |
| sodium chloride | 0.8 |
| ammonium sulfate | 0.5 |
| pH | natural |

a solvent is water.

In a preferred embodiment of the present disclosure, the step (3) comprises:
A. taking the *Saccharomyces* sp. OMK-76 refrigerated in glycerol, streaking on a plate, placing the plate at 27-28°C, and cultivating until clones are full;
B. picking a single clone from the clones, inoculating into a third seed cultivating medium, cultivating at 29-30 °C and 180-220 rpm on a shaker or stirring and cultivating at 380-420 rpm in an aeration condition for 20-25 hours to obtain a third seed cultivating solution;
C. inoculating the third seed cultivating solution into a third fermentation medium with an inoculum amount of 8-12%, fermenting for 8-12 hours at 29-30 °C and natural pH until OD₆₀₀ analyzed by a spectrophotometer no longer increases, adding p-hydroxybenzylidene acetone at one time until a final concentration is 48-52 g/L, continually cultivating until a substrate is completely reacted or no longer continues to be reacted, and then purifying to obtain the raspberry ketone.

In a further preferred embodiment, a composition of the third seed cultivating medium is shown in the following table:

| Product | Concentration (g/L) |
|---|---|
| yeast powder | 10 |
| peptone | 5 |
| glucose | 30 |
| potassium dihydrogen phosphate | 1.0 |
| sodium chloride | 0.8 |
| ammonium sulfate | 0.5 |
| pH | natural |

a solvent is water.

In a further preferred embodiment, a composition of the third fermentation medium is shown in the following table:

| Product | Concentration (g/L) |
|---|---|
| glucose | 100 |
| yeast powder | 10 |
| peptone | 0.5 |
| magnesium sulfate | 0.3 |
| potassium dihydrogen phosphate | 1.0 |
| sodium chloride | 0.8 |
| ammonium sulfate | 1.0 |
| pH | natural |

a solvent is water.

The present disclosure has the following advantages. The present disclosure discloses a highly effective method for preparing raspberry ketone using biological synthesis by a three-step fermentation. Step 1: natural p-coumaric acid is used as a raw material and is metabolied by microorganisms (*Actinomycetes* sp. OMK-74) to produce natural p-hydroxybenzaldehyde; Step 2: p-hydroxybenzaldehyde and acetone are metabolied by microorganisms (*Bacillus* sp. OMK-75) to produce 4-hydroxybenzylidene acetone; finally, 4-hydroxybenzylidene acetone is reduced by yeast (*Saccharomyces* sp.OMK-76) to specifically and efficiently produce raspberry ketone.

### BRIEF DESCRIPTION OF THE DRAWINGS

Fig. 1 illustrates a technical illustration of Embodiment 1 of the present disclosure.

### DETAILED DESCRIPTION OF THE EMBODIMENTS

The technical solution of the present disclosure will be further described in combination with the accompanying embodiments and drawings.

### Embodiment 1

Microorganisms used in this embodiment are isolated and screened from numerous microorganisms in marine soil, wherein *Actinomycetes* sp. OMK-74 was deposited in the China Center for Type Culture Collection on October 16, 2020, with a deposit number of CCTCC NO: M 2020584; *Bacillus* sp. OMK-75 was deposited in the China Center for Type Culture Collection on October 16, 2020, with a deposit number of CCTCC NO: M 2020585; and *Saccharomyces* sp. OMK-76 in 2020 was deposited in the China Center for Type Culture Collection on October 16, 2010, with a deposit number of CCTCC NO: M2020586. Referring to Fig. 1, a method for preparing raspberry ketone (i.e., frambione) by industrial fermentation comprises the following steps:
(1) Natural p-coumaric acid is used as a raw material and is metabolized by *Actinomycetes* sp. OMK-74 to produce p-hydroxybenzaldehyde, and which in particular comprises the following steps:
   A. A tube of *Actinomycetes* sp. OMK-74 that is refrigerated at 80 °C in glycerin and in refrigerator is streaked on a plate that is fresh and sterile, and the plate is placed in an incubator and is cultivated for 72 hours at 26 °C until clones are full;
   B. A single clone is picked from the clones, inoculated into a 3 L bioreactor having 1.8 L of a first seed cultivating medium, and stirred and cultivated for 24 hours at 37 °C and 300 rpm (revolutions per minute) under an aeration condition to obtain a first seed cultivating solution, which is used to be inoculated into a fermenter after no other bacteria is detected out by microscopic examination;
   C. The first seed cultivating solution is inoculated into a 20 L bioreactor having 10.2 L of a first fermentation medium (which is sterilized at 121 °C for 30 minutes) with an inoculum amount of 10% and fermented for 12 hours under conditions of 37 °C, pH=7.0-7.5 (which is automatically controlled by a 30% sodium hydroxide solution), a stirring speed of 400 rpm, and an aeration ratio of 1:0.2. When OD₆₀₀ analyzed by a spectrophotometer no longer increases, the p-coumaric acid is added at one time until a final concentration is 50 g/L (a total mount is 600 g), it is then continually cultivated for 48 hours until the substrate (i.e., the p-coumaric acid) is completely reacted or no longer continues to be reacted (which is sampled every 4 hours). When the fermentation is complete, a concentration of p-hydroxybenzaldehyde in the fermentation broth is analyzed by HPLC (High Performance Liquid Chromatography) and is 35g/L, and which is then purified to obtain the p-hydroxybenzaldehyde;

A composition of the first seed cultivating medium is shown in the following table:

| Product | Concentration (g/L) |
|---|---|
| malt extract powder | 30 15 |
| yeast powder | 1.2 |
| potassium dihydrogen phosphate | 0.8 |
| sodium chloride pH | Natural (e.g., without additional pH adjustment) |

A solvent is water.

A composition of the first fermentation medium is shown in the following table:

| Product | Concentration (g/L) |
|---|---|
| glucose | 30 |
| yeast powder | 0.8 |
| magnesium sulfate | 0.2 |
| potassium dihydrogen phosphate | 0.2 |
| sodium chloride | 0.25 |
| disodium phosphate | 0.5 |
| pH | Natural (e.g., without additional pH |

| | |
|---|---|
| | adjustment) |

A solvent is water.
(2) The p-hydroxybenzaldehyde obtained in step (1) and acetone are used as raw materials and are metabolized by *Bacillus* sp. OMK-75 to produce 4-hydroxybenzylidene acetone, and which in particular comprises the following steps:
A. A tube of the *Bacillus* sp. OMK-75 refrigerated at -80°C in glycerin and in a refrigerator is streaked on a plate that is fresh and sterile, and the plate is placed in a 30 °C incubator and is cultivated for 24 hours until clones are full;
B. A single clone is picked from the clones, inoculated into a 3 L bioreactor having 1.8 L of a second seed cultivating medium, and stirred and cultivated for 24 hours under an aeration condition at 30 °C and 400 rpm to obtain a second seed cultivating solution, which is used to be inoculated into a fermenter after no other bacteria is detected out by microscopic examination;
C. The second seed cultivating solution is inoculated into a bioreactor having 10.2 L of a second fermentation medium (which is sterilized at 121 °C for 30 minutes) with an inoculum amount of 10% and is fermented for 10 hours under conditions of 35°C, pH=6.5-7.0 (which is automatically controlled by a 30% sodium hydroxide solution), a stirring speed of 500rpm, and an aeration ratio of 1:0.3 until OD₆₀₀ analyzed by a spectrophotometer no longer increases, p-hydroxybenzaldehyde and acetone are added at one time until final concentrations are respectively 40 g/L (a total amount of 306 g) and 100 g/L, and it is then continually cultivated for 72 hours until the substrate is completely reacted or no longer continues to be reacted (which is sampled every 4 hours). After the fermentation is complete, a concentration of the p-hydroxybenzylidene acetone in the fermentation broth analyzed by HPLC is 50 g/L, and which is then purified to obtain the p-hydroxybenzylidene acetone.

A composition of the second seed cultivating medium is shown in the following table:

| Product | Concentration (g/L) |
|---|---|
| glucose | 20 10 |
| yeast powder | 10 |
| peptone | 0.8 |
| dipotassium phosphate | 0.8 |
| sodium chloride | Natural (e.g., without additional pH adjustment) |
| pH | |

A solvent is water.

A composition of the second fermentation medium is shown in the following table:

| Product | Concentration (g/L) |
|---|---|
| glucose | 30 8.0 |
| yeast powder | 2.0 |
| peptone | 0.2 |
| magnesium sulfate | 1.0 |
| potassium dihydrogen phosphate | 0.8 |
| sodium chloride | 0.5 |
| ammonium sulfate | natural (e.g., without additional pH adjustment) |
| pH | |

A solvent is water.
(3) The 4-hydroxybenzylidene acetone obtained in step (2) is used as a raw material and is metabolized by the *Saccharomyces* sp. OMK-76 and is reduced to produce the raspberry ketone, and which in particular comprises the following steps:
A. A tube of *Saccharomyces* sp. OMK-76 that is refrigerated in glycerin in a refrigerator at -80 °C is streaked on a plate that is fresh and sterile, and the plate is placed in a 28 °C incubator for 24 hours until clones are full;
B. A single clone is picked from the clones, inoculated into a 3 L bioreactor having 1.8 L of a third seed cultivating medium, and stirred and cultivated under an aeration condition at 30 °C and 400 rpm for 24 hours to obtain a third seed cultivating solution, which is used to be inoculated into a fermenter after no other bacteria is detected out by microscopic examination;
C. The third seed cultivating solution is inoculated into a bioreactor having 10.2 L of a second fermentation medium (which is sterilized at 121 °C for 30 minutes) with an inoculum amount of 10% and is fermented for 10 hours under conditions of 30 °C, natural pH, a stirring speed of 500 rpm, and an aeration ratio of 1:0.35 until OD₆₀₀ analyzed by a spectrophotometer no longer increases, p-hydroxybenzylidene acetone that a final concentration is 40 g/L (a total amount of 480 g) and glucose that a final concentration is 100 g/L are added at one time, and it is then continually incubated for 72 hours until the substrate is completely reacted or no longer continues to be reacted (which is sampled every 4 hours). When the fermentation is complete, a concentration of the raspberry ketone in the fermentation broth analyzed by HPLC is 39.5 g/L, and which is then purified to obtain the raspberry ketone.

A composition of the third seed cultivating medium is shown in the following table:

| Product | Concentration (g/L) |
|---|---|
| | 10 |
| yeast powder | 5 |
| peptone | 30 |
| glucose | 1.0 |
| potassium dihydrogen phosphate | 0.8 |
| sodium chloride | 0.5 |
| ammonium sulfate pH | natural (e.g., without additional pH adjustment) |

A solvent is water.

A composition of the third fermentation medium is shown in the following table:

| Product | Concentration (g/L) |
|---|---|
| glucose | 100 |
| yeast power | 10 |
| peptone | 0.5 |
| magnesium sulfate | 0.3 |
| potassium dihydrogen phosphate | 1.0 |
| sodium chloride | 0.8 |
| ammonium sulfate | 1.0 |
| pH | natural (e.g., without additional pH adjustment) |

A solvent is water.

The invention may be summarized as follows: The present disclosure is a method for preparing raspberry ketone using industrial fermentation, which comprises the following steps:
(1) using natural p-coumaric acid as a raw material, metabolizing by Actinomycetes sp. OMK-74 to obtain p-hydroxybenzaldehyde; (2) using the p-hydroxybenzaldehyde obtained in step (1) and acetone as raw materials, metabolizing by Bacillus sp. OMK-75 to obtain 4-hydroxybenzyl acetone; and (3) using the 4-hydroxybenzyl acetone obtained in step (2) as a raw material, metabolizing by Saccharomyces sp. OMK-76 to be reduced to obtain the raspberry ketone. The present disclosure discloses a highly effective method for preparing raspberry ketone using biological synthesis by a three-step fermentation.

The aforementioned embodiments are merely some embodiments of the present disclosure, and the scope of the disclosure is not limited thereto. Thus, it is intended that the present disclosure cover any modifications and variations of the presently presented embodiments provided they are made without departing from the appended claims and the specification of the present disclosure.

## Claims

1. A method for preparing raspberry ketone using industrial fermentation, **characterized in that**, which comprises the following steps:
(1) using natural p-coumaric acid as a raw material, metabolizing by *Actinomycetes* sp. OMK-74 to obtain p-hydroxybenzaldehyde;
(2) using the p-hydroxybenzaldehyde obtained in step (1) and acetone as raw materials, metabolizing by *Bacillus* sp. OMK-75 to obtain 4-hydroxybenzyl acetone; and
(3) using the 4-hydroxybenzyl acetone obtained in step (2) as a raw material, metabolizing by *Saccharomyces* sp. OMK-76 to be reduced to obtain the raspberry ketone;
*Actinomycetes* sp. OMK-74 was deposited in China Center for Type Culture Collection on October 16, 2020, with a deposit number of CCTCC NO: M 2020584; *Bacillus* sp. OMK-75 was deposited in China Center for Type Culture Collection on October 16, 2020, with a deposit number of CCTCC NO: M 2020585; and *Saccharomyces* sp. OMK-76 was deposited in China Center for Type Culture Collection on October 16, 2020, with a deposit number of CCTCC NO: M2020586.

2. The method using the industrial fermentation according to claim 1, **characterized in that**, the step (1) comprises:
A. taking *Actinomycetes* sp. OMK-74 refrigerated in glycerin, streaking on a plate, placing the plate at 36-37 °C, and cultivating until clones are full;
B. picking a single clone from the clones, inoculating into a first seed cultivating medium, cultivating at 36-37 °C and 180-220 rpm on a shaker or stirring and cultivating at 280-320 rpm in an aeration condition for 20-25 hours to obtain a first seed cultivating solution;
C. inoculating the first seed cultivating solution in a first fermentation medium with an inoculum amount of 8-12%, fermenting for 12-16 hours at 36-37 °C and pH=7.0-7.5 until OD₆₀₀ analyzed by a spectrophotometer no longer increases, adding p-coumaric acid at one time or several times until a final concentration is 48-52 g/L, then continually cultivating until a substrate is completely reacted or no longer continues to be reacted, and then purifying to obtain the p-hydroxybenzaldehyde.

3. The method using the industrial fermentation according to claim 2, **characterized in that**, a composition of the first seed cultivating medium is shown in the following table:
| Product | Concentration (g/L) |
|---|---|
| malt extract powder | 30 |
| yeast powder | 15 |
| potassium dihydrogen phosphate | 1.2 |
| sodium chloride | 0.8 |
| pH | natural |
a solvent is water.

4. The method using the industrial fermentation according to claim 2 and/or 3, **characterized in that**, a composition of the first fermentation medium is shown in the following table:
| Product | Concentration (g/L) |
|---|---|
| glucose | 30 |
| yeast powder | 0.8 |
| magnesium sulfate | 0.2 |
| potassium dihydrogen phosphate | 0.2 |
| sodium chloride | 0.25 |
| disodium phosphate | 0.5 |
| pH | natural |
a solvent is water.

5. The method using the industrial fermentation of any one or more of claims 1 to 4, **characterized in that**, the step (2) comprises:
A. taking the *Bacillus* sp. OMK-75 refrigerated in glycerin, streaking on a plate, placing the plate at 29-30 °C, and cultivating until clones are full;
B. picking a single clone from the clones, inoculating into a second seed cultivating medium, cultivating at 29-30 °C and 180-220 rpm on a shaker or stirring and cultivating at 380-420 rpm in an aeration condition for 20-25 hours to obtain a second seed cultivating solution;
C. inoculating the second seed cultivating solution into a second fermentation medium with an inoculum amount of 8-12%, fermenting for 8-12 hours at 34-35 °C and pH=6.5-7.0 until OD₆₀₀ analyzed by a spectrophotometer no longer increases, adding p-hydroxybenzaldehyde and acetone at one time until final concentrations are respectively 38-42 g/L and 100-150 g/L, then continually cultivating until a substrate is completely reacted or no longer continues to be reacted, and then purifying to obtain the p-hydroxybenzylidene acetone.

6. The method using the industrial fermentation according to claim 5, **characterized in that**, a composition of the second seed cultivating medium is shown in the following table:
| Product | Concentration (g/L) |
|---|---|
| glucose | 20 |
| yeast powder | 10 |
| peptone | 10 |
| dipotassium phosphate | 0.8 |
| sodium chloride | 0.8 |
| pH | natural |
a solvent is water.

7. The method using the industrial fermentation according to claim 5 and/or 6, **characterized in that**, a composition of the second fermentation medium is shown in the following table:
| Product | Concentration (g/L) |
|---|---|
| glucose | 30 |
| yeast powder | 8.0 |
| peptone | 2.0 |
| magnesium sulfate | 0.2 |
| potassium dihydrogen phosphate | 1.0 |
| sodium chloride | 0.8 |
| ammonium sulfate | 0.5 |
| pH | natural |
a solvent is water.

8. The method using the industrial fermentation according to any one or more of claims 1 to 7, **characterized in that**, the step (3) comprises:
A. taking the *Saccharomyces* sp. OMK-76 refrigerated in glycerol, streaking on a plate, placing the plate at 27-28°C, and cultivating until clones are full;
B. picking a single clone from the clones, inoculating into a third seed cultivating medium, cultivating at 29-30 °C and 180-220 rpm on a shaker or stirring and cultivating at 380-420 rpm in an aeration condition for 20-25 hours to obtain a third seed cultivating solution;
C. inoculating the third seed cultivating solution into a third fermentation medium with an inoculum amount of 8-12%, fermenting for 8-12 hours at 29-30 °C and natural pH until OD₆₀₀ analyzed by a spectrophotometer no longer increases, adding p-hydroxybenzylidene acetone at one time until a final concentration is 48-52 g/L, continually cultivating until a substrate is completely reacted or no longer continues to be reacted, and then purifying to obtain the raspberry ketone.

9. The method using the industrial fermentation according to claim 8, **characterized in that**, a composition of the third seed cultivating medium is shown in the following table:
| Product | Concentration (g/L) |
|---|---|
| yeast powder | 10 |
| peptone | 5 |
| glucose | 30 |
| potassium dihydrogen phosphate | 1.0 |
| sodium chloride | 0.8 |
| ammonium sulfate | 0.5 |
| pH | natural |
a solvent is water.

10. The method using the industrial fermentation according to claim 8 and/or 9, **characterized in that**, a composition of the third fermentation medium is shown in the following table:
| Product | Concentration (g/L) |
|---|---|
| glucose | 100 |
| yeast powder | 10 |
| peptone | 0.5 |
| magnesium sulfate | 0.3 |
| potassium dihydrogen phosphate | 1.0 |
| sodium chloride | 0.8 |
| ammonium sulfate | 1.0 |
| pH | natural |
a solvent is water.
